# EUROPEAN PATENT APPLICATION

(11) **EP 3 170 494 A2**
(43) Date of publication of application: **24.05.2017**
(21) Application number: 16201764.4
(22) Date of filing: 21.11.2008
(51) Int. Cl.: A61K 31/135, A61K 31/137, A61K 31/192, A61K 31/197, A61P 29/00, A61K 9/24

(54) **TAPENTADOL COMPOSITIONS**

(30) Priority: 23.11.2007 US 4029 P
(62) Divisional of application: 13000915.2
(71) Applicant: Grünenthal GmbH, 52078 Aachen (DE)
(72) Inventor: SESHA, Ramesh, West Windsor, NJ New Jersey 08550 (US)
(74) Representative: Kutzenberger Wolff & Partner

(57) **Abstract**

The present invention provides a method of treating pain and pain related conditions by administering to a patient in need thereof, a therapeutically effective amount of a slow release Tapentadol Hydrochloride and therapeutically effective amount of a second analgesic, wherein the second analgesic is tramadol, gamma-aminobutyric acid (GABA) analogue or an NSAID. The present invention further provides a pharmaceutical composition comprising a therapeutically effective amount of a slow release Tapentadol Hydrochloride and a therapeutically effective amount of a second analgesic, wherein the second analgesic is tramadol, gamma-aminobutyric acid (GABA) analogue or an NSAID.

## Description

### Related Applications

This application claims priority from a U.S. provisional patent application serial no. 61/004,029 filed on November 23, 2007, which is incorporated herein by reference

### Background of the Invention

Tapentadol, 3-(3-Dimethylamino-1-ethyl-2-methyl-propyl)-phenol (compound 1) is a centrally acting analgesic with a dual mode of action: µ-opioid receptor agonism and noradrenalinne reuptake inhibition. Its dual mode of action provides analgesia at similar levels of more potent narcotic analgesics such as hydrocodone, oxycodone, and morphine with a more tolerable side effect profile. Tapentadol was first disclosed in European patent no. EP 693,475 and is currently under FDA review.

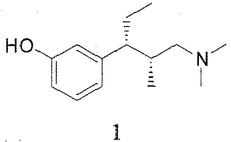

The traditional formulations of tapentadol for oral administration lead to a rapid release of the drug in the gastrointestinal tract and hence its analgesic action begins rapidly. However, a rapid reduction in the analgesic activity is observed. Therefore, treatment with tapentadol requires repeated administration of the pharmaceutical composition at relatively short intervals, often as high as four to ten times daily, to maintain the required concentration of active ingredient in the patient's blood plasma. The need for repeated dosing can lead to errors in administration and inability to maintain desirable concentration in the plasma, which are detrimental to patient compliance and the therapeutic objectives, particularly if the condition is chronic pain or a pain related condition. Accordingly, there is an unmet need to have slow or controlled release pharmaceutical compositions for oral administration of the active ingredient, tapentadol. Further, there is a need for compositions suitable for long term treatment of pain and pain related conditions particularly because such conditions persist among aged populations of the society.

Pregabalin (compound 2), a gamma-aminobutyric acid (GABA) analogue, is an anticonvulsant drug which is used as an adjunct therapy for partial seizures, for neuropathic pain, and in generalized anxiety disorder. Pregabalin was designed as a more potent successor to gabapentin and it is marketed by Pfizer under the trade name Lyrica®. Recent studies have shown that pregabalin is effective at treating chronic pain in disorders such as fibromyalgia and spinal cord injury.

Gabapentin (compound 3) is another GABA analogue similar to Pregabalin and was initially synthesized to mimic the chemical structure of the neurotransmitter gamma-aminobutyric acid (GABA), but is not believed to act on the same brain receptors. Its exact mechanism of action is unknown, but its therapeutic action on neuropathic pain is thought to involve voltage-gated N-type calcium ion channels.

Most anti-inflammatory drugs such as non- steroidal anti inflammatory drugs (NSAIDs) have been associated with an increased risk of serious upper gastrointestinal complications. The risk is believed to be dose dependent and can be greater when more than one anti-inflammatory drug is administered. Hence, whenever possible, anti-inflammatory drugs should be administered in mono-therapy. This risk can be more pronounced in case of non-aspirin non- steroidal anti inflammatory (NA-NSAID) drugs. Intake of NA-NSAIDs as a group has been consistently associated with a four- to five-fold increase in upper gastrointestinal complications (UGIC). The evidence indicates that the risk is dose dependent. The estimated pooled cardiovascular Relative Risks (RR) in a recent meta-analysis were 3.0 (95% CI, 2.6-3.4) for low doses, 4.1 (95% CI, 3.6-4.5) for medium doses, and 6.9 (95% CI, 5.8-8.1) for high doses. Recent research indicates that NA-NSAID as a therapeutic class have a RR of 4.1 (95% CI, 3.6-4.8).

Meloxicam (compound 4), an oxicam derivative, is a member of the enolic acid group of non-steroidal anti-inflammatory drugs (NSAIDs). It is reported to be a selective COX-2 inhibitor. Meloxicam is chemically known as 4-hydroxy-2-methyl-N-(5-methyl-2-thiazolyl)-2H-1,2-benzothiazine-3-carboxamide-1,1-dioxide. It is commercially available under the trade name of MOBIC®. Meloxicam is indicated for relief of the signs and symptoms of osteoarthritis and rheumatoid arthritis, pauciarticular or polyarticular course Juvenile Rheumatoid Arthritis in patients 2 years of age and older.

Naproxen (compound 5) is another non-steroidal anti-inflammatory drug (NSAID) commonly used for the reduction of mild to moderate pain, fever, inflammation and stiffness caused by conditions such as osteoarthritis, rheumatoid arthritis, psoriatic arthritis, gout, ankylosing spondylitis, injury (like fractures), menstrual cramps, tendonitis, bursitis, and the treatment of primary dysmenorrhea. Naproxen, chemically known as (+)-(S)-2-(6-methoxynaphthalen-2-yl) propanoic acid, and naproxen sodium are marketed under various trade names including: Aleve, Anaprox, Naprogesic, Naprosyn, Naprelan, and Synflex. There have also been reports of naproxen producing disturbances in the gastrointestinal tract, like other NSAIDs. Additional NSAIDs include but are not limited to compound 6, Diclofenac; compound 7, Celecoxib; compound 8, Diflunisal; compound 9, Etodolac; compound 10, Fenoprofen; compound 11, Ibuprofen; compound 12, Indomethacin; compound 13, Ketoprofen, and compound 14, Ketorolac.

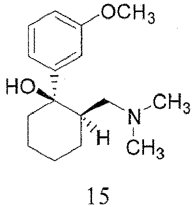

Tramadol (compound 15) is a centrally acting synthetic opioid analgesic. It is chemically (±) cis-2-[(dimethylamino) methyl)-1-(3-methoxyphenyl) cyclo-hexanol hydrochloride. A commercially available form is the hydrochloride salt as Ultram tablets. Tramadol has been used for the management of moderate to moderately severe pain in adults. Tramadol is a non-NSAID analgesic that is not believed to cause the increased risk of stomach ulceration and internal bleeding associated with non-steroidal anti inflammatory drugs (NSAID). However, it still has some commonly reported side effects including nausea, constipation, dizziness, headache, drowsiness, and vomiting. Other reported side effects include itching, sweating, dry mouth, diarrhea, rash, visual disturbances, and vertigo. Thus, it would be desirable to prevent or reduce these side effects by prescribing lower doses of tramadol without compromising pain relief.

Literature reports indicate that NSAIDs such as naproxen can inhibit the excretion of sodium and lithium. Hence it is desirable to control their dosage to alleviate side effects in patients without comprising the extent of pain relief. Similarly, dizziness, somnolence, dry mouth, edema, blurred vision, weight gain, and "thinking abnormal" (primarily difficulty with concentration/attention) were more commonly reported by subjects treated with GABA analogues like pregabalin.

There is a strong unmet medical need for drugs that are free from side effects associated with, tramadol, pregabalin and NSAIDs. Considering that such drugs are often used over a long term by elderly patients to manage pain that is often chronic, compositions that can help reduce the dosage or frequency of either or both of drug types without comprising the therapeutic benefits would fill this medical unmet need.

Opioids have been combined with other drugs including non-opioid analgesic agents, to try to lower the amount of opioid needed to produce an equivalent degree of analgesia and reduce the side effects from opioids. It has been reported that some of these combination products also have a synergistic analgesic effect. See for example, A. Takemori, Annals New York Acad. Sci., 281,262 (1976) where compositions including combinations of opioid analgesics with non-analgesic drugs are reported to exhibit a variety of effects, e.g., sub additive (inhibitory), additive or super additive. Also, R. Taber et al., J. Pharm. Expt. Thera., 169(1), 29 (1969) describes a combination of morphine and methadone, another opioid analgesic. U.S. Patent No. 4,571,400 discloses a combination of dihydrocodeine, an opioid analgesic, and ibuprofen, a non-opioid analgesic. See also U.S. Patent Nos. 4,587,252 and 4,569,937, which disclose other ibuprofen opioid combinations. A. Pircio et al., Arch. Int. Pharmacodyn., 235, 116 (1978) report a 1:125 mixture of butorphanol, another opioid analgesic, and acetaminophen, a non-opioid analgesic has a greater effect than a 1:10 mixture.

Combinations of non-opioid analgesics have also been prepared to avoid the side effects associated with opioids, and the combinations are noted to have the benefit of requiring less of each ingredient and may provide additive effects. See, *e.g.* G. Stacher et al., Int. J. Clin. Pharmacol. Biopharmacy, 17, 250 (1979), U.S. Patent. No. 4,260,629, U.S. Patent No. 4,132,788. However, there have been warnings against the daily consumption of non-opioid analgesic mixtures and of the consumption of a single non-opioid analgesic in large amounts or over long periods (see, D. Woodbury and E. Fingl at page 349). In addition, ibuprofen, aspirin and some other NSAIDs may cause gastrointestinal side effects especially if used repeatedly. See, *e.g.,* M. J. S. Langman, Am. J. Med. 84 (Suppl. 2A): 15-19, 1988); P. A. Insel in "Goodman and Gilman's The Pharmacological Basis of Therapeutics. " Gilman AG, Rall TW, Nies AS, et al. (eds). Pergamon Press, 8th Ed, 1990, Chapter 26, pp. 664-668.

Neuropathic pain is believed to be caused by a primary lesion or dysfunction in the nervous system. Neuropathic pains have been categorized as peripheral neuropathic pain, due to lesion of the peripheral nervous system and central pain following lesions of the central nervous system. The prevalence of neuropathic pain is estimated to be about 1%. Neuropathic pain has been shown to be therapy resistant. However, a number of agents have been used to treat neuropathic pain including NSAIDs, opioids, antidepressants, anticonvulsants, excitatory amino acid antagonists, GABAergic agonists, Substance P antagonists, etc. Low doses of carbamazepine and amitriptyline have been recommended for neuropathic pain in general. The side effects of GABA agonists such as gabapentin, pregabalin, etc. have been documented in the literature. Hence, it is desirable to reduce their dosage to alleviate the patients of its side effects without comprising the extent of pain relief.

A number of treatments involving the administration of single drugs are currently recommended for pain relief. The single administration of narcotic and non-narcotic analgesics and NSAIDs has been shown to display pain alleviating properties. Some anti-epileptics, such as gabapentin and pregabalin, have also been reported to have pain alleviating properties in diabetic neuropathy.

Despite the benefits derived from current single drug pain relief regimens, these regimens have disadvantages. There is a strong unmet medical need for drugs that are free from side effects associated with tapentadol, tramadol, GABA analogue or NSAIDs. One area of concern relates to the incidence of unwanted side effects caused by many of the pain treatment regimens available today. Opioid analgesics, such as morphine, are sparingly prescribed for pain because of the well-known addictive effects and significant central nervous system (CNS) side effects and gastrointestinal side effects. Further tapentadol is known to elicit adverse effects, including nausea, vomiting, sleepiness, dizziness, itchiness, sedation, dry mouth, sweating and constipation.

However, a pharmaceutical composition comprising a slow release tapentadol and a second analgesic, wherein the second analgesic is tramadol, gamma-aminobutyric acid (GABA) analogue or an NSAID for treating a patient in need there of. Further, the prior art doesn't disclose a method of treating pain or pain related disorder comprising a method of administering to a mammal in need thereof, a pharmaceutical composition comprising a slow release tapentadol and a second analgesic, wherein the second analgesic is tramadol, gamma-aminobutyric acid (GABA) analogue or an NSAID is not disclosed. As there is a continuing need for analgesic medications that provide high efficacy pain relief with reduced undesirable effects.

### Summary

The present invention provides a pharmaceutical combination comprising a slow release tapentadol and a second analgesic agent. The second analgesic agent can be tramadol, gamma-aminobutyric acid (GABA) analogue or an NSAID. The invention further provides a method for treating pain and pain related disorders in a mammal, comprising administering to said mammal an effective amount of a composition comprising a slow release tapentadol and a second analgesic agent.

In another embodiment the invention provides a tapentadol / analgesic combination for treating moderate to severe painful conditions associated with diabetic neuropathy, rheumatoid arthritis, osteoarthritis and the like, by administering to a subject in need thereof, an analgesic pharmaceutical combination comprising from about 25 to about 400 mg of slow (controlled) release tapentadol and a second analgesic agent, wherein the second analgesic agent is about 5 to about 500 mg of tramadol hydrochloride, about 5 to about 500 mg of a GABA agonist, or about 5 to about 500 mg of an NSAID with pharmaceutically acceptable carrier so as to provide better pain management. In the pharmaceutical composition, the tapentadol is in a controlled release form and tramadol hydrochloride, a GABA analogue, or an NSAID are present in an immediate release form, extended (controlled) release form or delayed release form along with pharmaceutically acceptable carrier.

An advantage of the disclosed compositions is a decreased dosing of the active ingredients, such as tramadol, GABA analogue or NSAIDs to the patient which can promote better patient compliance. Further, the compositions comprising from about 25 to about 400 mg of slow release tapentadol and a second analgesic agent, wherein the second analgesic is tramadol hydrochloride, a GABA agonist, or an NSAID with pharmaceutically acceptable carrier so as to provide better pain management

### Brief Description of the Figures

FIGURE 1 illustrates a comparison of the in vitro dissolution profiles of tapentadol HCl in slow release tapentadol HCl 100 mg and naproxen 250 mg tablets.
FIGURE 2 illustrates a comparison of the LS mean change from baseline in VAS score for the combination drug comprising slow release tapentadol 100 mg and naproxen 250 mg with those of tapentadol and naproxen mono therapies based upon the average of Weeks 1-6.
FIGURE 3 illustrates a weekly LS mean changes from baseline for the four treatment groups.
FIGURE 4 illustrates a mean VAS pain score changes for four formulations; tapentadol 100 MG, pregabalin 250 MG, and slow release tapentadol 100 MG + pregabalin 250 MG fixed dose combination.
FIGURE 5 illustrates a mean VAS pain score changes for three formulations; Tramadol 50 mg, Tapentadol 100 MG, Placebo and Slow Release Tapentadol 100 MG + Tramadol 50 MG fixed dose combination.
FIGURE 6 illustrates a mean VAS pain score changes for the four formulations; tapentadol 100 MG, gabapentin 250 MG, and slow release tapentadol 100 plus gabapentin 250 MG fixed dose combination.

### Detailed Description

One object of the present invention is to provide methods, which can be used in the treatment of pain and pain related diseases wherein the methods comprise administration of a therapeutically effective amount of a slow release tapentadol and a second analgesic agent, wherein the second analgesic agent is tramadol, gamma-aminobutyric acid (GABA) analogue or an NSAID to a patient in need thereof.

The two analgesic agents *e.g.* a slow release tapentadol and a second analgesic agent may be co-administered in a single medicament or they may be administered separately as two medicaments. Further, the first drug may (tapentadol) be administered in a regimen, which additionally comprises administration of the second drug separately or in a composition with the first drug.

In yet another embodiment, the invention provides a slow release tapentadol and a second analgesic agent are administered in suboptimal dosages.

In yet another embodiment, the invention provides a slow release tapentadol and a second analgesic agent are administered in amounts and for a sufficient time to produce a synergistic effect.

In yet another embodiment, the invention provides a composition where the second active agent is included in an immediate release coating.

In yet another embodiment, the invention provides a bilayer composition where one layer includes the tapentadol and one layer comprises the second active agent.

In yet another embodiment, there is provided a method of treating moderate to severe pain by administering to a subject in need thereof, a pharmaceutical composition comprising 5-500 mg tapentadol or a second analgesic, wherein the second analgesic is from about 5 to about 500 mg of tramadol hydrochloride, from about 5 to about 500 mg of a GABA agonist, or from about 5 to about 500 mg of an NSAID thereof in admixture with pharmaceutically acceptable carrier.

Still further, a titration dosing regimen for the administration of slow release tapentadol to patients. The titration dosing regimen provides a significant reduction in the occurrence of adverse effects from the introduction of slow release tapentadol dosing, thus increasing patient compliance and medication tolerability.

In yet another embodiment, the invention provides a composition of tapentadol and a second analgesic agent, or a pharmaceutically acceptable salts thereof for use in medical treatment (for example, treatment of pain, e.g., neuropathic pain).

In yet another embodiment, the invention provides a method for the use of tapentadol and a second analgesic agent, or a pharmaceutically acceptable salts thereof to prepare a medicament for treatment of pain in a mammalian species (for example, a human).

### DETAILED DESCRIPTION OF THE INVENTION

The term "Analgesic" as used in this invention means to include any drug used to relieve pain including paracetamol (acetaminophen), the non-steroidal anti-inflammatory drugs (NSAIDs) such as the salicylates, narcotic drugs such as morphine, synthetic drugs with narcotic properties such as tramadol, GABA analogues like pregabalin, gabapentin and various others other classes of drugs not normally considered analgesics are used to treat neuropathic pain syndromes; these include tricyclic antidepressants and anticonvulsants

The term "band range" for purposes of the present invention is defined as the difference in *in vitro* dissolution measurements of the controlled release formulations when comparing the dissolution profile (curve) obtained by the formulation upon completion of the manufacturing of the coated product (prior to storage) and the dissolution profile obtained after the coated product is exposed to accelerated storage conditions, expressed as the change in percent of the active agent released from the coated product at any dissolution time point along the dissolution curves.

The term "co-administration" as used herein means administration of the two drugs (agents) together (*e*.*g*., simultaneously as a mixture) or administration can be sequential. The sequential administration of the tapentadol can be prior to or after administration of the second analgesic agent, within minutes of each other or up to about 48 hours after the administration of the other agent. Preferably the administration of the tapentadol will be within about 24 hours of administration of the second analgesic agent and more preferably within about 12 hours of administration of the second analgesic agent.

The term "effective amount" as used herein means a dosage to produce a selected effect. For example, an effective amount of an analgesic is an amount which is sufficient in order for the pain of the patient to be reduced when compared with no treatment.

The term "GABA analogue" as used in this invention means any analogue of the mammalian neurotransmitter gamma-aminobutyric acid (GABA) that inhibit the release of several neurotransmitters such as glutamate, noradrenaline, and substance P. Non-limiting examples of GABA analogues include pregabalin, gabapentin, their pharmaceutically equivalent salts, isomers, polymorphs, hydrates, complexes or clatharates and the like.

The term "NSAID" as used in this specification means any non-steroidal anti-inflammatory drug. Non-limiting examples include Celecoxib, Diclofenac, Diflunisal, Etodolac, Fenoprofen, Flurbirofen, Ibuprofen, Indomethacin, Ketoprofen, Ketorolac, Mefenamic acid, Meloxicam, Nabumetone, Naproxen, Oxaprozin, Piroxicam, Sulindac and Tolmetin and their pharmaceutically equivalent salts, isomers, polymorphs, hydrates, complexes, or clatharates and the like. Non-limiting examples of the GABA analogues are gabapentin, pregabalin or a pharmaceutically acceptable salt thereof.

In yet another embodiment, the invention provides a composition where the second active agent is included in an immediate release coating. The pharmaceutical composition of any of claims 1-5, wherein the composition exhibits an in vitro dissolution profile such that after 2 hours, from about 0% to about 30% by weight of tapentadol is released, after 4 hours, from about 5% to about 22% by weight of tapentadol is released, after 6 hours, from about 15% to about 30% by weight of tapentadol is released, and after 8 hours, more than about 40% by weight of tapentadol is released; when measured using the USP Basket Method at 75 rpm in 900 ml 0.1 N HCl at 37° C.

The term "medicament" as used herein means a pharmaceutical composition suitable for administration of the pharmaceutically active compound to a patient.

The term "pharmaceutically-acceptable salt" refers to salts that retain the biological effectiveness and properties of the disclosed compounds and which are not biologically or otherwise undesirable. In many cases, the disclosed compounds are capable of forming acid or base salts by virtue of the presence of amino or carboxyl groups or groups similar thereto. The preparation of the salts and suitable acids or bases is known in the art.

The term "suboptimal dosage" us used herein means a dosage which is below the optimal dosage for that compound when used in single-compound therapy.

The term "additive effect" as used herein means the effect resulting from the sum of the effects obtained from the individual compounds.

The term "synergistic effect" as used herein means an effect which is greater than the additive effect which results from the sum of the effects of the two individual compounds.

The term "treatment of a disease" as used herein means the management and care of a patient having developed the disease, condition or disorder. The purpose of treatment is to combat the disease, condition or disorder. Treatment includes the administration of the active compounds to eliminate or control the disease, condition or disorder as well as to alleviate the symptoms or complications associated with the disease, condition or disorder.

The term "prevention of a disease" as used herein is defined as the management and care of an individual at risk of developing the disease prior to the clinical onset of the disease. The purpose of prevention is to combat the development of the disease, condition or disorder, and includes the administration of the active compounds to prevent or delay the onset of the symptoms or complications and to prevent or delay the development of related diseases, conditions or disorders.

The term "pain and pain related conditions" as used herein is defined as any pain due to a medical condition including neuropathic pain, osteoarthritis, rheumatoid arthritis, fibromyalgia, and back, musculoskeletal pain, Ankylosing spondylitis, juvenile rheumatoid arthritis, migraines, dental pain, abdominal pains, ischemic pain, postoperative pain or because of an anesthetic or surgical contrition

The term "extended release material" as present in the inner solid particulate phase or the outer solid continuous phase refers to one or more hydrophilic polymers and/or one or more hydrophobic polymers and/or one or more other type hydrophobic materials, such as, for example, one or more waxes, fatty alcohols and/or fatty acid esters. The "extended release material" present in the inner solid particulate phase may be the same as or different from the "extended release material" present in the outer solid continuous phase.

The term "slow-release" or "controlled release" as used herein applies to any release from a formulation that is other than an immediate release wherein the release of the active ingredient is slow in nature. This includes various terms used interchangeably in the pharmaceutical context like extended release, delayed release, sustained release, controlled release, timed release, specific release and targeted release etc.

The term "candidate for sustained release" encompasses all the characteristics of a drug which make it a candidate for formulating it into an extended release fashion like a short elimination half life and consequent dosing of more than once a day, a single dose product given in an extended fashion to achieve better clinical results and avoid side effects associated with an immediate release etc.

The term "binding agent" as used in this specification, refers to any conventionally known pharmaceutically acceptable binder such as polyvinyl pyrrolidone, hydroxypropyl cellulose, hydroxyethyl cellulose, hydroxypropyl methylcellulose, ethylcellulose, polymethacrylate, polyvinylalcohol, waxes and the like. Mixtures of the aforementioned binding agents may also be used. The preferred binding agents are water soluble materials such as polyvinyl pyrrolidone having a weight average molecular weight of 25,000 to 3,000,000. The binding agent may comprise approximately about 0 to about 40% of the total weight of the core and preferably about 3% to about 15% of the total weight of the core. In one embodiment, the use of a binding agent in the core is optional.

The term "pharmaceutically acceptable derivative" means various pharmaceutical equivalent isomers, enantiomers, complexes, salts, hydrates, polymorphs, esters etc of tapentadol or tramadol or a GABA analogue or an NSAID.

The term "therapeutically effective amount" means an amount that elicits a biological response in a mammal including the suboptimal amount.

The term "hydrophilic polymers" as used in this specification include, but are not limited, to hydroxypropylmethylcellulose, hydroxypropylcellulose, sodium, carboxymethyl- cellulose, carboxymethylcellulose calcium, ammonium alginate, sodium alginate, potassium alginate, calcium alginate, propylene glycol alginate, alginic acid, polyvinyl alcohol, povidone, carbomer, potassium pectate, potassium pectinate, etc.

The term "hydrophobic polymers" as used in this specification include, but are not limited, to ethyl cellulose, hydroxyethylcellulose, ammonio methacrylate copolymer (Eudragit™ RL or Eudragit™ RS), methacrylic acid copolymers (Eudragit™ L or Eudragit™ S), methacrylic acid-acrylic acid ethyl ester copolymer (Eudragit™ L 100-5), methacrylic acid esters neutral copolymer (Eudragit™ NE 30D), dimethylaminoethylmethacrylate-methacrylic acid esters copolymer (Eudragit™ E 100), vinyl methyl ether/malefic anhydride copolymers, their salts and esters (Gantrez™) etc.

Other hydrophobic materials which may be employed in the inner solid particulate phase and/or outer solid continuous phase include, but are not limited, to waxes such as beeswax, carnauba wax, microcrystalline wax, and ozokerite; fatty alcohols such as cetostearyl alcohol, stearyl alcohol; cetyl alcohol myristyl alcohol etc; and fatty acid esters such as glyceryl monostearate, glycerol monooleate, acetylated monoglycerides, tristearin, tripalmitin, cetyl esters wax, glyceryl palmitostearate, glyceryl behenate, hydrogenated castor oil, etc.

Non-limiting examples of NSAIDs for the compositions include Celecoxib, Diclofenac, Diflunisal, Etodolac, Fenoprofen, Flurbirofen, Ibuprofen, Indomethacin, Ketoprofen, Ketorolac, Mefenamic acid, Meloxicam, Nabumetone, Naproxen, Oxaprozin, Piroxicam, Sulindac and Tolmetin and their pharmaceutically equivalent salts, isomers, polymorphs, hydrates, complexes, or clatharates and the like. Non-limiting examples of the GABA analogues are gabapentin, pregabalin or a pharmaceutically acceptable salt thereof.

The disclosed pharmaceutical compositions can exhibit an in vitro dissolution profile such that after 2 hours, from about 0% to about 30% by weight of tapentadol is released, after 4 hours, from about 5% to about 22% by weight of tapentadol is released, after 6 hours, from about 15% to about 30% by weight of tapentadol is released, and after 8 hours, more than about 40% by weight of tapentadol is released; when measured using the USP Basket Method at 75 rpm in 900 ml 0.1 N HCl at 37° C.

The tramadol material is any one of (1R, 2R or 1S, 2S)-(dimethyl aminomethyl)-1-(3-methoxyphenyl)-cyclo-hexanol (tramadol), its N-oxide derivative ("tramadol N-oxide"), and its O-desmethyl derivative ("O-desmethyl tramadol") or mixtures thereof. It also includes the individual stereoisomer, mixtures of stereoisomer, including the racemates, pharmaceutically acceptable salts of the amines, such as the hydrochloride salt, solvates and polymorphs of the tramadol material. Tramadol is commercially available from Grunenthal or may be made by the process described in U.S. Pat. No. 3,652,589 and it is herein incorporated by reference.

A combination comprising a slow release tapentadol and a second analgesic agent, wherein the second analgesic agent is tramadol, gamma-aminobutyric acid (GABA) analogue or an NSAID. The disclosed compositions preferably contain a therapeutically effective amount of tapentadol or a pharmaceutically acceptable salt thereof, wherein the tapentadol is in the range of from about 5 to about 800 mg, preferably from about 50, to about 600 mg, more preferably from about 100 to about 400 mg and more preferably from about 200 to about 300 mg (calculated as tapentadol hydrochloride) per dosage unit and a therapeutically effective amount of a second analgesic agent, wherein the second analgesic is from about 5 to about 500 mg of tramadol, from about 5 to about 500 mg of tramadol gamma-aminobutyric acid (GABA) analogue and from about 5 to about 500 mg of tramadol NSAID.

The disclosed composition can be, for example, as granules, spheroids, pellets, multiparticulates, capsules, patches tablets, sachets, controlled release suspensions, or in any other suitable dosage form incorporating such granules, spheroids, pellets or multiparticulates.

The compositions can be, *e.g*., coated tablets wherein the coating includes at least one water-insoluble, water permeable film-forming polymer, at least one plasticizer and at least one water-soluble polymer, and the second active agent. In a preferred form the coating can have at least one water-insoluble, water-permeable film-forming polymer varies from about 20% to about 90% of the coating dry weight, the proportion of the at least one plasticizer varies from about 5% to about 30% of the coating dry weight, and the proportion of the at least one water-soluble polymer varies from about 10% to about 75% of the coat dry weight.

A preferred water-insoluble, water-permeable film-forming polymer is ethylcellulose. A preferred water-insoluble polymer is polyvinylpyrrolidone. A preferred plasticizer is dibutyl sebacate.

The one or more of active ingredient in the combination according to the present invention may suitably be incorporated in a matrix. This may be any matrix, known to a person skilled the art, that affords slow release tapentadol over at least about a twelve hour period and preferably that affords in-vitro dissolution rates and in vivo absorption rates of tapentadol within the therapeutically effective ranges. The combination according to the present invention may preferably use a slow release matrix. Alternatively, normal release matrices having a coating which provides for slow release of the tapentadol may be used.

The slow release matrix employed in the combination of this invention may also contain other pharmaceutically acceptable ingredients which are conventional in the pharmaceutical art such as diluents, lubricants, binders, granulating aids, colorants, flavorants, surfactants, pH adjusters, anti-adherents and glidants, e.g., dibutyl sebacate, ammonium hydroxide, oleic acid and colloidal silica. Any known diluent e.g. microcrystalline cellulose, lactose and dicalcium phosphate may be used to prepare this combination. Suitable lubricants are e.g. magnesium stearate and sodium stearyl fumarate. Suitable binding agents are e.g. hydroxypropyl methyl cellulose, polyvidone and methyl cellulose. Suitable disintegrating agents are starch, sodium starch glycolate, crospovidone, and croscarmellose sodium.

The surface actives that are suitable for this invention are Poloxamer 188.RTM, polysorbate 80 and sodium lauryl sulfate. The suitable flow aids for this invention are talc colloidal anhydrous silica. Non-limiting water soluble polymers that may be used to prepare the matrix include PEG having weight average molecular weights in the range of from about 1000 to about 6000. The combination comprising the slow release tapentadol according to the invention may conveniently be film coated using any film coating material conventional in the pharmaceutical art but preferably an aqueous film coating is used.

Alternatively, the combination comprising a slow release tapentadol and a second analgesic, wherein the second analgesic is tramadol, gamma-aminobutyric acid (GABA) analogue or an NSAID as per this invention may comprise a normal release matrix having a slow release coating. Preferably the combination comprises film coated spheroids containing the active ingredient and a spheronising agent. The spheronising agent may be any suitable pharmaceutically acceptable material which may be spheronised together with the active ingredient to form spheroids. A preferred spheronising agent as per this invention is microcrystalline cellulose. The microcrystalline cellulose used may suitably be, for example, Avicel™ PH 101 or Avicel™ PH 102 (FMC Corporation). The spheroids may optionally contain other pharmaceutically acceptable ingredients conventional in the pharmaceutical art such as binders, bulking agents and colorants. Suitable binders may include water soluble polymers, water soluble hydroxyalkyl celluloses such as hydroxypropylcellulose or water insoluble polymers (which may also contribute controlled release properties) such as acrylic polymers or copolymers for example ethylcellulose. Suitable bulking agents include lactose.

The spheroids are coated with a material which permits release of the active ingredient at a slow rate in an aqueous medium. Suitable slow release coating materials that may be used in this invention include water insoluble waxes and polymers such as polymethylacrylates (for example Eudragit™ polymers) or water insoluble celluloses, particularly ethylcellulose. Optionally, water soluble polymers such as polyvinylpyrrolidone or water soluble celluloses such as hydroxypropylmethylcellulose or hydroxypropylcellulose may be included. Optionally other water soluble agents such as polysorbate 80 may be added.

Further in an alternative embodiment, a flux-enhancing agent can also be included in the membrane or slow release coating can include one of the above-described polymers. The flux enhancing agent can increase the volume of fluid imbibed into the core to enable the dosage form to dispense substantially all of the tapentadol through the passage and/or the porous membrane. The flux-enhancing agent can be a water-soluble material or an enteric material. Examples of the preferred materials that are useful as flux enhancers include but not limited to sodium chloride, potassium chloride, sucrose, sorbitol, mannitol, polyethylene glycols (PEG), propylene glycol, hydroxypropyl cellulose, hydroxypropyl methycellulose, hydroxypropyl methycellulose phthalate, cellulose acetate phthalate, polyvinyl alcohols, methacrylic acid copolymers, poloxamers (such as LUTROL™ F68, LUTROL F127, LUTROL F108 which are commercially available from BASF) and mixtures thereof. A preferred flux-enhancer used in this invention is PEG 400.

The flux enhancer may also be a water miscible/soluble drug such as Tapentadol or its pharmaceutically acceptable salts, or the flux enhancer may be a drug that is soluble under intestinal conditions. If the flux enhancer is a drug, the present pharmaceutical composition has an added advantage of providing an immediate release of the drug that has been selected as the flux enhancer. The flux enhancing agent dissolves or leaches from the membrane or sustained release coating to form channels in the membrane or sustained release coating which enables fluid to enter the core and dissolve the active ingredient. In the preferred embodiment, the flux enhancing agent comprises approximately 0 to about 40% of the total weight of the coating, most preferably from about 2% to about 20% of the total weight of the coating.

A commonly known excipient such as a plasticizer may also be used for preparing the membrane or slow release coating Some commonly known plasticizers include but not limited to adipate, azelate, enzoate, citrate, stearate, isoebucate, sebacate, triethyl citrate, tri-n-butyl citrate, acetyl tri-n-butyl citrate, citric acid esters, and all those described in the Encyclopedia of Polymer Science and Technology, Vol. 10 (1969), published by John Wiley & Sons. The preferred plasticizers are triacetin, acetylated monoglyceride, grape seed oil, olive oil, sesame oil, acetyltributylcitrate, acetyltriethylcitrate, glycerin sorbitol, diethyloxalate, diethylmalate, diethylfumarate, dibutylsuccinate, diethylmalonate, dioctylphthalate, dibutylsebacate, triethylcitrate, tributylcitrate, glyceroltributyrate and the like. Though the exact amount used depends on the type of plasticizer used, typically amounts from 0 to about 25% are used, and preferably from about 2% to about 15% of the plasticizer can be used based upon the total weight of the membrane or sustained release coating.

Generally, the membrane or slow release coating around the core will comprise from about 1% to about 20% and preferably about 2% to about 10% based upon the total weight of the core and coating.

The membrane or sustained release coating surrounding the core can further comprise a passage that will allow for controlled release of the drug from the core in a preferred embodiment. As used herein the term passage includes an aperture, orifice, bore, hole, weakened area or a credible element such as a gelatin plug that erodes to form an osmotic passage for the release of the tapentadol from the dosage form. Passage used in accordance with the subject invention is well known and are described in U.S. Pat. Nos. 3,845,770; 3,916,899; 4,034,758; 4,077,407; 4,783,337 and 5,071,607.

The following examples 1-13 are shown for illustrating the invention related to combination comprising a slow release tapentadol and a second analgesic, wherein the second analgesic is tramadol, gamma-aminobutyric acid (GABA) analogue or an NSAID. According to this invention where we have used a tramadol hydrochloride, specific GABA analogues Pregabalin and gabapentin specific NSAIDs Meloxicam and Naproxen only as examples for illustrative purposes and these examples in no way limit the scope of the invention. The person skilled in the art will know how the combination may be modified using other GABA analogues or other NSAIDs such as Celecoxib, Diclofenac, Diflunisal, Etodolac, Fenoprofen, Flurbirofen, Ibuprofen, Indomethacin, Ketoprofen, Ketorolac, Mefenamic Acid, Meloxicam, Nabumetone, Naproxen, Oxaprozin, Piroxicam, Sulindac and Tolmetin and using other manufacturing methods known in the art.

### Example 1:

**TABLE 1**

| Combination of slow release tapentadol 100 mg and naproxen 250 mg tablets | |
|---|---|
| **First Active Ingredient mg/tablet** | Example 1 |
| Tapentadol Hydrochloride | 100.0 |
| Microcrystalline Cellulose | 10.0 |
| Colloidal Silicon Dioxide | 1.5 |
| Polyvinylpyrrolidone | 4.5 |
| Hydrogenated Vegetable Oil | 5.0 |
| Water* | Q.S |

| **Coat** | |
|---|---|
| Ethylcellulose Aqueous Dispersion | 15.00 |
| Polyvinylpyrrolidone | 5.0 |
| Polyethylene Glycol | 2.0 |
| Water* | Q.S |

| **Second Active Ingredient** | |
|---|---|
| Naproxen | 250.0 |
| Povidone K 30 USP | 12 |
| Microcrystalline cellulose | 25 |
| Croscarmellose sodium | 15 |
| Magnesium Stearate | 3 |
| Water* | Q.S. |

| | |
|---|---|
| * Removed during processing | |

### Manufacturing Process

The combination comprising a slow release tapentadol hydrochloride tablets and naproxen were manufactured in two phases using standard coating processes. In phase I, the Tapentadol Hydrochloride was formulated into a core that was further coated with slow release coat to get a slow release tapentadol core. In Phase II, this slow release coated Tapentadol hydrochloride core was coated with an immediate release layer comprising Naproxen as per details are given below;
**Phase I, Core preparation:** Tapentadol HCl is mixed with microcrystalline cellulose and colloidal silicone dioxide and one or mixture of filler and granulated using suitable method known in the art using a binder solution comprising Polyvinylpyrrolidone or polyvinyl alcohol. The granulated tapentadol hydrochloride was dried and screened. This is further lubricated using hydrogenated vegetable oil with or without glidant. The lubricated blend is compressed into tablets using a compression machine.

**Coating Solution and Coating:** The coating solution is prepared using aqueous dispersion of water insoluble water permeable polymer of Ethylcellulose with water soluble polymer of Polyvinylpyrrolidone or hydroxypropyl methyl cellulose. Polyethylene glycol mixture prepared using propeller stirrer and the same is homogenized using suitable homogenizer. The core tablets are coated using coating solution using standard coater like O' Hara pan coater tip set at 4" at a spray rate of 25 mL/gun/min, exhaust temperature of around 45'C, an atomization pressure from 10-35 psi at a pan speed of 5-8 rpm, using airflow 350 CFM.

**Phase II:** In phase II, Naproxen formulation prepared using granulation technique known in the art and then blended with disintegrant and lubricant. Tapentadol slow release tablets prepared in Phase I is coated with lubricated blend of Naproxen formulation using compression coating machine where Tapentadol slow release tablets is used as a core and an immediate layer of Naproxen formulation forms an outer layer.

The naproxen coating was applied to slow release coated 100 mg tapentadol hydrochloride tablets using the above mentioned coater. Over this naproxen coated seal coated 100 mg tapentadol hydrochloride tablets, color coating was done using similar coat. The spraying was done at a temperature of 46-47' C, atomization pressure of 40-60 psi at a spray rate of 180 grams per minute/three guns. The pan speed was at 4-8 rpm and air volume of 1000±100.

Finally, color coated tablets were dried and optionally polished using Cindrella wax and the finished final tablets were packaged in a HDPE bottle with a suitable desiccant and subjected appropriate stability and clinical studies. In Example 1, a pharmaceutical composition comprising 100 mg of slow release tapentadol and 250 mg naproxen was formulated as per Table 1.

The manufacturing processes exemplified here are only meant for illustration and the pharmaceutical composition can be prepared using a number of methods well established in the art. Other exemplified formulations are listed below in tables 2-5;

### Example 2:

**TABLE 2**

| Combination of slow release tapentadol 100 mg and naproxen 250 mg tablets | |
|---|---|
| **First Active Ingredient mg/tablet** | **Quantity mg** |
| Tapentadol HCl | 100.0 |
| Polyvinyl Alcohol | 2.0 |
| Colloidal Silicon Dioxide (Abrosil™ 200) | 1.0 |
| Sodium Stearyl Fumarate | 1.0 |
| Water* | Q.S |
| Core Weight | 104.0 |

| **Coat** | |
|---|---|
| Ethylcellulose (Ethocel™ PR 100) | 9.20 |
| Polyvinylpyrrolidone (Kollidon™ 90F) | 4.14 |
| Dibutyl Sebacate | 2.66 |
| Denatured Alcohol* | Q.S |

| **Second Active Ingredient mg/tablet** | |
|---|---|
| Naproxen | 250.0 |
| Povidone K 30 USP | 12 |
| Microcrystalline cellulose | 25 |
| Croscarmellose sodium | 15 |
| Magnesium Stearate | 3 |
| Water* | Q.S. |

| | |
|---|---|
| * Removed during processing | |

### Example 3

**TABLE 3**

| Combination of slow release tapentadol 100 mg and naproxen 250 mg tablets | |
|---|---|
| **First Active Ingredient mg/tablet** | **Quantity mg** |
| Tapentadol HCl | 100.0 |
| Polyvinyl Alcohol | 2.0 |
| Colloidal Silicon Dioxide (Abrosil™ 200) | 1.0 |
| Sodium Stearyl Fumarate | 1.0 |
| Water* | Q.S |
| Core Weight | 104.0 |

| **Coat** | |
|---|---|
| Ethylcellulose (Ethocel™ PR 100) | 9.87 |
| Polyvinylpyrrolidone (Kollidon™ 90F) | 3.47 |
| Dibutyl Sebacate | 2.67 |
| Denatured Alcohol* | Q.S |

| **Second Active Ingredient mg/tablet** | |
|---|---|
| Naproxen | 250.0 |
| Povidone K 30 USP | 12 |
| Microcrystalline cellulose | 25 |
| Croscarmellose sodium | 15 |
| Magnesium Stearate | 3 |
| Water* | Q.S. |

| | |
|---|---|
| * Removed during processing | |

### Example 4

**TABLE 4**

| Combination of slow release tapentadol 100 mg and naproxen 250 mg tablets | |
|---|---|
| **First Active Ingredient mg/tablet** | **Quantity mg** |
| Tapentadol HCl | 100.0 |
| Polyvinyl Alcohol | 2.0 |
| Colloidal Silicon Dioxide (Abrosil® 200) | 1.0 |
| Sodium Stearyl Fumerate | 1.0 |
| Water* | Q.S |
| Core Weight | 104.0 |

| **Coat** | |
|---|---|
| Ethylcellulose (Ethocel® PR 100) | 9.87 |
| Polyvinylpyrrolidone (Kollidon® 90F) | 3.73 |
| Dibutyl Sebacate | 2.67 |
| Denatured Alcohol* | Q.S |

| **Second Active Ingredient mg/tablet** | |
|---|---|
| Naproxen | 250.0 |
| Povidone K 30 USP | 12 |
| Microcrystalline cellulose | 25 |
| Croscarmellose sodium | 15 |
| Magnesium Stearate | 3 |
| Water* | Q.S. |

| | |
|---|---|
| * Removed during processing | |

### Example 5

**TABLE 5**

| Combination of slow release tapentadol 200 mg and naproxen 500 mg tablets | |
|---|---|
| **First Active Ingredient mg/tablet** | **Quantity mg** |
| Tapentadol HCl | 200.0 |
| Polyvinyl Alcohol | 4.0 |
| Colloidal Silicon Dioxide (Abrosil™ 200) | 2.0 |
| Sodium Stearyl Fumarate | 2.0 |
| Water* | Q.S |
| Core Weight | 2.8 |

| **Coat** | |
|---|---|
| Ethylcellulose (Ethocel™ PR 100) | 12.28 |
| Polyvinylpyrrolidone (Kollidon™ 90F) | 6.50 |
| Dibutyl Sebacate | 3,75 |
| Denatured Alcohol* | Q.S |

| **Second Active Ingredient mg/tablet** | |
|---|---|
| Naproxen | 500.0 |
| Povidone K 30 USP | 20 |
| Microcrystalline cellulose | 40 |
| Croscarmellose sodium | 25 |
| Magnesium Stearate | 6 |
| Water* | Q.S. |

| | |
|---|---|
| * Removed during the process | |

**Core Preparation;** Tapentadol HCl and colloidal silicon dioxide were mixed and passed through a 1.0 mm screen. Polyvinyl alcohol was dissolved in purified water. The mixed tapentadol HCl and colloidal silicon dioxide powder was granulated with the aqueous solution of polyvinyl alcohol in a fluidized bed granulator, Glatt GPCGl and then dried. After granulation, the granules were blended with sodium stearyl fumarate and then passed through a 1.0 mm screen. The blend was then compressed into tablet cores using a Manesty Betapress.

**Coating Preparation;** The ethyl alcohol and isopropanol were weighed and mixed. Dibutyl sebacate and ethylcellulose were added to and dissolved in the ethyl alcohol and isopropyl alcohol while stirring using a propeller stirrer, Coframo RZR1. The ethylcellulose and dibutyl sebacate were allowed to dissolve completely. The polyvinylpyrrolidone was added. The solution was stirred until all components were dissolved. The solution was passed through a high pressure homogenizer, Mini DeBee 2000 with a #7 nozzle, Bee International. The tablet cores were coated using the coating solution in a perforated coating pan, O'Hara Labcoat I11 36" Pan, Vector LCDS. The coating parameters are listed in Table 6;

**Table 6**

| Coating Parameters | |
|---|---|
| Inlet Temperature: | 48.5-49.5' C |
| Outlet Temperature: | 38.5-39.5' C |
| Bed Temperature: | 37.5-38.5' C |
| Spray Rate: | 300g/minute |
| Atomizing Air/ Pattern: | 25/25 psi |
| Distance gun/Bed: | 6" |
| Distance between guns: | 6" |
| Pan speed: | 12rpm |

| Coating Amount Diameter: | |
|---|---|
| Thickness: | 6 mm |
| Cup Height: | 4.65 mm |
| Surface: | 1.02 mm |
| Percentage: | 112 mm² |
| Amount: | 16 mg |

### Example 6

In yet another example, the invention discloses a pharmaceutical composition which can effectively be used in the treatment of pain and pain related diseases wherein the compositions comprise a therapeutically effective amount of a slow release tapentadol and an NSAID to a patient in need can be formulated in other ways. For Example, the combination comprising a slow release tapentadol and an NSAID such as Naproxen was prepared as a bilayer tablet as exemplified below:

**Layer 1:**

| | |
|---|---|
| Tapentadol HCl | 200 mg |
| Microcrystalline cellulose | 10 - 25% |
| Polyvinyl alcohol | 3 - 5% |
| Ethylcellulose (5- 20 cp) | 10 - 20% |
| Hydroxyethyl cellulose | 5 - 15% |
| Colloidal silicon dioxide | 2 - 5% |
| Sodium stearyl fumarate | 1 - 2% |

**Layer 2:**

| | |
|---|---|
| Naproxen | 250 mg |
| Microcrystalline cellulose | 5-20% |
| Povidone | 10 -15% |
| Crosscarmellose sodium | 5 - 10% |
| Magnesium stearate | 0.5 - 2% |

Preparation of Layer 1: Tapentadol Hydrochloride, microcrystalline cellulose and colloidal silicon dioxide were granulated with polyvinyl alcohol and dried. The dried granules are mixed with Ethylcellulose and Hydroxyethylcellulose and lubricated with Sodium stearyl fumarate.

Preparation of Layer 2: Naproxen mixed with microcrystalline cellulose was granulated with povidone. Granules are dried and mixed with Crosscarmellose sodium and finally lubricated with Magnesium stearate.

Compression: Layer 1 and Layer 2 are loaded into the hopper of Bilayer rotary compression machine and compressed with a desired hardness.

### Example 7

**Table 7**

| **Meloxicam Combination** | |
|---|---|
| **First Active Ingredient mg/tablet** | |
| Tapentadol Hydrochloride | 100.0 |
| Lactose | 65.0 |
| Ethyl Cellulose | 16.0 |
| Cetostearyl Alcohol | 43.0 |
| Magnesium Stearate | 2.0 |
| Talc | 4.0 |
| Hydroxyethyl Cellulose | |
| Water * | qs |

| **Coat** | |
|---|---|
| Hydroxypropyl methyl cellulose | 0.75 |
| Hydroxy methyl cellulose | 3.75 |
| Opaspray | 2.60 |
| PEG 400 | 0.60 |
| Talc | 0.30 |
| Water * | qs |

| **Second Active Ingredient** | |
|---|---|
| Meloxicam | 7.5 |
| Povidone K 30 USP | 1.0 |
| Lactose | 25.0 |
| Sodium starch Glycolate | 7.5 |
| Poloxamer 188 | 3.0 |
| HPMC | 1.5 |
| PEG 8000 | 0.4 |
| Titanium Dioxide | 0.4 |
| Wax | 0.2 |
| * Removed during processing | |

### Example 8

**Table 8**

| **Meloxicam Combination** | |
|---|---|
| **First Active Ingredient mg/tablet** | |
| Tapentadol Hydrochloride | 100.0 |
| Lactose | 66.0 |
| Ethyl Cellulose | 0.0 |
| Cetostearyl Alcohol | 44.0 |
| Magnesium Stearate | 2.0 |
| Talc | 4.0 |
| Hydroxyethyl Cellulose | 14.0 |
| Water * | qs |

| **Coat** | |
|---|---|
| Hydroxypropyl methyl cellulose | 0.75 |
| Hydroxy methyl cellulose | 3.75 |
| Opaspray | 2.60 |
| PEG 400 | 0.60 |
| Talc | 0.30 |
| Water * | qs |

| **Second Active Ingredient** | |
|---|---|
| Meloxicam | 7.5 |
| Povidone K 30 USP | 1.0 |
| Lactose | 25.0 |
| Sodium starch Glycolate | 7.5 |
| Poloxamer 188 | 3.0 |
| HPMC | 1.5 |
| PEG 8000 | 0.4 |
| Titanium Dioxide | 0.4 |
| Wax | 0.2 |
| * Removed during processing | |

### Manufacturing Process, Slow release tapentadol hydrochloride and meloxicam

The combination comprising a slow release tapentadol hydrochloride tablets and meloxicam were manufactured using standard granulation and coating processes. Tapentadol hydrochloride and lactose were granulated together in a granulator and sprayed with ethylcellulose and water. The granulated tapentadol hydrochloride was dried and screened. The tapentadol hydrochloride granules were mixed with Cetostearyl alcohol. Talc and magnesium stearate were mixed with the tapentadol hydrochloride and the granules were compressed into tablets. The compressed tablets were coated using the coating constituents The above prepared coated slow release tapentadol tablets were further seal coated with Opadry Clear (YS-1-7006) solution using standard coater like O' Hara pan coater tip set at 4" at a spray rate of 25 mL/gun/min, exhaust temperature of around 45'C, an atomization pressure from 10-35 psi at a pan speed of 5-8 rpm, using airflow 350 CFM. The meloxicam coating was applied to coated 100 mg tapentadol hydrochloride tablets using the above mentioned coater. Over this 7.5 mg meloxicam coated seal coated 100 mg tapentadol hydrochloride tablets, color coating was done using similar coat. The spraying was done at a temperature of 46-47' C, atomization pressure of 40-60 psi at a spray rate of 180 grams per minute/three guns. The pan speed was at 4-8 rpm and air volume of 1000±100.

Finally, color coated tablets were dried and polished using Cindrella wax and the finished final tablets were packaged in a HDPE bottle with a suitable desiccant and subjected appropriate stability and clinical studies.

### Example 9

**Table 9**

| **Pregabalin Combination** | |
|---|---|
| **First Active Ingredient mg/tablet** | |
| Tapentadol Hydrochloride | 100.0 |
| Lactose | 65.0 |
| Ethyl Cellulose | 16.0 |
| Cetostearyl Alcohol | 43.0 |
| Magnesium Stearate | 2.0 |
| Talc | 4.0 |
| Hydroxyethyl Cellulose | |
| Water * | qs |

| **Coat** | |
|---|---|
| Hydroxypropyl methyl cellulose | 0.75 |
| Hydroxy methyl cellulose | 3.75 |
| Opaspray | 2.60 |
| PEG 400 | 0.60 |
| Talc | 0.30 |
| Water * | qs |

| **Second Active Ingredient** | |
|---|---|
| Pregabalin | 250 |
| Povidone K 30 USP | 1.0 |
| Lactose | 25.0 |
| Sodium starch Glycolate | 7.5 |
| Poloxamer 188 | 3.0 |
| HPMC | 1.5 |
| PEG 8000 | 0.4 |
| Titanium Dioxide | 0.4 |
| Wax | 0.2 |
| * Removed during processing | |

### Example 10

**Table 10**

| **Pregabalin Combination** | |
|---|---|
| **First Active Ingredient mg/tablet** | |
| Tapentadol HCl | 200.0 |
| Polyvinyl Alcohol | 4.0 |
| Colloidal Silicon Dioxide (Abrosil® 200) | 2.0 |
| Sodium Stearyl Fumarate | 2.0 |
| Water* | Q.S |
| Core Weight | 2.8 |

| **Coat** | |
|---|---|
| Ethylcellulose (Ethocel® PR 100) | 12.28 |
| Polyvinylpyrrolidone (Kollidon® 90F) | 6.50 |
| Dibutyl Sebacate | 3,75 |
| Denatured Alcohol* | Q.S |

| **Second Active Ingredient** | |
|---|---|
| Pregabalin | 250 mg |
| Povidone K 30 USP | 12 mg |
| Microcrystalline cellulose | 25 mg |
| Croscarmellose sodium | 15 mg |
| Magnesium Stearate | 3 mg |
| Water* | Q.S. |
| * Removed during processing | |

### Example 11

**Table 11**

| **Gabapentin Combination** | |
|---|---|
| **First Active Ingredient mg/tablet** | |
| Tapentadol Hydrochloride | 100.0 |
| Lactose | 65.0 |
| Ethyl Cellulose | 16.0 |
| Cetostearyl Alcohol | 43.0 |
| Magnesium Stearate | 2.0 |
| Talc | 4.0 |
| Hydroxyethyl Cellulose | |
| Water * | Qs |

| **Coat** | |
|---|---|
| Hydroxypropyl methyl cellulose | 0.75 |
| Hydroxy methyl cellulose | 3.75 |
| Opaspray | 2.60 |
| PEG 400 | 0.60 |
| Talc | 0.30 |
| Water * | qs |

| **Second Active Ingredient** | |
|---|---|
| Gabapentin | 250 |
| Povidone K 30 USP | 1.0 |
| Lactose | 25.0 |
| Sodium starch Glycolate | 7.5 |
| Poloxamer 188 | 3.0 |
| HPMC | 1.5 |
| PEG 8000 | 0.4 |
| Titanium Dioxide | 0.4 |
| Wax | 0.2 |
| * Removed during processing | |

### Example 12

**Table 12**

| **Pregabalin Combination** | |
|---|---|
| **First Active Ingredient mg/tablet** | |
| Tapentadol HCl | 200.0 |
| Polyvinyl Alcohol | 4.0 |
| Colloidal Silicon Dioxide (Abrosil® 200) | 2.0 |
| Sodium Stearyl Fumarate | 2.0 |
| Water* | Q.S |
| Core Weight | 2.8 |

| **Coat** | |
|---|---|
| Ethylcellulose (Ethocel® PR 100) | 12.28 |
| Polyvinylpyrrolidone (Kollidon® 90F) | 6.50 |
| Dibutyl Sebacate Denatured Alcohol* | 3,75 Q.S |

| **Second Active Ingredient** | |
|---|---|
| Pregabalin | 250 mg |
| Povidone K 30 USP | 12 mg |
| Microcrystalline cellulose | 25 mg |
| Croscarmellose sodium | 15 mg |
| Magnesium Stearate | 3 mg |
| Water* | Q.S. |
| * Removed during processing | |

### Example 13

**Table 13**

| **Tramadol Combination** | |
|---|---|
| **First Active Ingredient mg/tablet** | |
| Tapentadol Hydrochloride | 100 |
| Microcrystalline Cellulose | 10 |
| Colloidal Silicon Dioxide | 1.5 |
| Polyvinylpyrrolidone | 4.5 |
| Hydrogenated Vegetable Oil | 5 |
| Water * | Q.S |

| **Coat** | |
|---|---|
| Ethylcellulose Aqueous Dispersion | 50 |
| Opaspray YS -1 7006 | 5 |
| Water* | Q.S |

| **Second Active Ingredient** | |
|---|---|
| Tramadol | 100 |
| Povidone K 30 USP | 10 |
| Microcrystalline cellulose | 15 |
| Croscarmellose sodium | 9 |
| Magnesium Stearate | 2 |
| Water* | Q.S |
| * Removed during processing | |

The disclosed compositions can also be prepared using methods that are well established in the art. It can be prepared using a general preparation outlined in tables 14and 15

**Table 14**

| **Formula** | | |
|---|---|---|
| First Active Ingredient | Range percent | Preferred Range % |
| Drug | 50-98% | 75-95% |
| Binder | 0.1-40% | 3-15% |
| Absorption Enhancer | 0-20% | 2-10% |
| Lubricant | 0-5% | 0.5-1% |
| Coat | | |
| Polymer | 50-99% | 75-95% |
| Flux Enhancer | 0-40% | 2-20% |
| Plasticizer | 0-25% | 2-15% |
| Second Active Ingredient | | |
| Drug | 0.1-20% | 1-10% |
| Binder | 0.1-20% | 1-15% |
| Surfactant | 0-20% | 0.1-15% |
| Pore Former | 0-25% | 0.1-15% |
| Polymer (Optional) | 0-30% | 0.1-20% |

**Table 15**

| **First Active Ingredient** | **Percent of Core** |
|---|---|
| Tapentadol HCl | 90.54% |
| Povidone K 301 USP | 4.38% |
| Sodium Tribasic Phosphate | 4.58% |
| Magnesium Stearate | 0.50% |

| **Membrane** | **Percent of membrane** |
|---|---|
| Cellulose Acetate (398-10)' 85% Triacetin 5% PEG 400 10% | 85.00% |
| Triacetin | 5.00% |
| PEG 400 | 10.00% |

| **Second Active Ingredient** | **Percent of second layer** |
|---|---|
| Second Analgesic | 43.50% |
| Tween | 2.00% |
| HPMC | 54.50% |

### METHOD OF ADMINISTRATION

The present inventions further include a method of treating pain and pain related conditions. This was established using well controlled human clinical trials for each type of combination. A typical study determined the efficacy of combination comprising a slow release tapentadol and Pregabalin, a combination comprising a slow release tapentadol and naproxen (NSAID) and a combination comprising a slow release tapentadol and tramadol. Each of these combinations was compared against monotherapy with the respective drugs for the treatment of pain and pain related conditions in patients.

### Clinical Trials:

The following general methods were used in all of the studies;

Randomization: Randomization was performed with computer-generated random numbers in blocks of 10. Randomization codes of the monotherapy or combination therapy treatments were placed in sequentially numbered, opaque, sealed envelopes in the biopsy center. When a patient was recruited and consented, the next numbered envelope was opened by the operator, who had no knowledge of the randomization code before the treatment

Scale and Measurement: Pain was assessed using a visual analogue scale (VAS) graded from 0-100 mm. Pain scoring was performed every visit Week 1, Week 2, Week 3, Week 4, Week 5 and Week 6. The patients were taught with a standard method by the physician how to use a VAS scoring from 0 to 100 mm to grade the intensity of pain experienced during the treatment. On that scale, the left endpoint, 0, was defined as no pain and the right endpoint 100, as the worst pain the patient could imagine. There were no further marks on the line. The intensity of pain was indicated by the distance in millimeters from the left end. Patients were asked to grade the pain intensity after the administration of drugs and the pain was assessed by the patient in their weekly pain score diary. The doctors who prescribed the medicines obtained all the VAS forms for that specific patient.

Statistical Analysis: Data are expressed as means ± SD. Differences in VAS were analyzed with use of the unpaired Student t test and differences in pain score reductions between the two groups were analyzed with use of the Mann-Whitney U test for the median difference. Similar statistical tests were used for between group comparisons of other outcome variables as appropriate. A two tailed P value of less than. 0.5 was considered to demonstrate statistical significance. SAS software was used for the statistical analysis.

### Study I, Tapentadol and NSAID Combination;

Patients: The efficacy was established using a 6-week, multi-center, double blind, randomized, parallel, three arm comparison of the efficacy of fixed dose slow release Tapentadol ER and Naproxen tablets, Tapentadol and Naproxen tablets in the treatment of knee osteoarthritis. The study enrolled 50 patients, from 18 to 75 years of age with moderate to severe pain associated with Functional Class 1-111 osteoarthritis. The enrollment was designed in such a way that a minimum of 30 patients completed the study in each arm. The patients who met the inclusion and exclusion criteria at screening, after signing the informed consent, entered a 7- day washout period where all analgesics were discontinued. During the visit 2 at the start of the first week of the study, the eligible patients who reported pain intensity 240 mm on a visual analog scale (VAS) in the index knee joint, had their VAS Baseline score measured and were randomly assigned to one of the three arms: The fixed dose tapentadol ER and Naproxen or to Tapentadol arm or to Naproxen arm. Table 16 shows the patient's profile of the study.

**Table 16**

| **Patient Characteristics** | | | | |
|---|---|---|---|---|
| **Characteristic** | **Treatment A** | **Treatment B** | **Treatment C** | **Placebo** |
| | **N= 30** | **N=30** | **N=34** | **N=30** |
| Mean age +/- SD (y) | 42.2 ±13 | 44.4±11 | 43.2 ± 12 | 41.2 ± 12 |
| Sex (M/F) | 17/13 | 15/15 | 18/16 | 16/14 |

Drugs and Treatment Arms: The treatment arms and drug regimen is listed below.

| **Treatment Arm** | **Drugs per day per patient** |
|---|---|
| 1. Treatment A; | Placebo |
| 2. Treatment B; | Tapentadol HCl 100 mg X 2 |
| 3. Treatment C: | Naproxen 250 mg X 2 |
| 4. Treatment D: | Combination of tapentadol HCl 200 mg + naproxen 500 MG. |

Patients were assigned to either placebo or tapentadol HCl 100 mg or naproxen 250 mg or fixed dose combination of tapentadol 100 mg plus naproxen 250 mg. The patients with pain unresponsive to treatments or with unacceptable side effects were discontinued. The patients returned for efficacy measurement on Week 1, Week 2, Week 3, Week 4, Week 5 and Week 6.

The primary measure of efficacy was the Arthritis Pain Intensity VAS (visual analog scale) Score from patient visits. The arthritis VAS is the most commonly used, validated tool to assess pain intensity and one recommended by FDA to evaluate the analgesic potential of a drug product.

We considered that a clinically significant benefit of using fixed dose slow release tapentadol HCl and naproxen would be a reduction in the pain score (VAS) of at least 15% compared to the monotherapy with either tapentadol hydrochloride or naproxen. Alternatively, the dose reduction of at least 15% of either naproxen or tapentadol, when used as a co-administered combination, over the monotherapy is considered as a significant benefit.

The objectives of the inventions are met by the following results from the clinical trials. A total of 206 patients were randomized and evaluable for safety. Of these, 170 were evaluable for the intent- to-treat (ITT) population. The ITT population included all safety evaluable patients who had primary efficacy information recorded at the baseline visit (Visit 2) and at the Week 1 visit (Visit 3), the first primary efficacy variable collection point on treatment. The ITT population also included all patients who dropped out before the Week 1 visit due to lack of treatment efficacy. The median age of patients who enrolled was 61 years and the median duration of osteoarthritis was 10 years.

Fixed dose combination comprising a slow release tapentadol and naproxen produced statistically significant and clinically meaningful reductions, compared to the monotherapy using either tapentadol or naproxen, for the primary efficacy variable in pain intensity associated with knee osteoarthritis.

### Tapentadol and GABA Analogue Combination, Study II

The study was of double-blind, randomized, placebo-controlled, and two-period cross-over design. After a 2-week run-in period, 32 diabetic patients (17 men, 15 women with type 2 diabetes, age [mean ± SE] 61.7 ± 1.6 years, duration of diabetes 8,8 ± 1.5 years, duration of painful neuropathy 2.2 ± 0.4 years) were randomized to receive either placebo or tapentadol hydrochloride 100 mg or pregabalin 250 mg/gabapentin 250 mg or slow release tapentadol HCl 100 mg + pregabalin 250 mg or gabapentin 250 mg FDC for 4 weeks, exchanging their treatment for a further 4 weeks after a 2-week wash-out period. The patients administered the spray to both feet before bedtime. Biweekly pain and other sensory symptoms were assessed using a visual analog scale (VAS). The patient characteristics are shown in Table 17.

**Table 17**

| Patient Characteristics | |
|---|---|
| Number of patients | 22 |
| Age (years) | 63.7±1.8 (41-76)* |
| Sex | 13 males, 9 females |
| BMI (kg/m2) | 32.8±1.4 |
| Type of diabetes | 2 type 1, 20 type 2 |
| Duration of diabetes (years) | 9.1±1.5 |
| Duration of neuropathy (years) | 3.0±0.5 |
| Duration of neuropathic pain (years) | 2.5±0.4 |
| Treatment order | 10 ISDN, 12 placebo |
| HbA1c (%)† | |
| At study entry | 7.8±0.3 |
| At study completion | 8.1±0.4 |

| | |
|---|---|
| *Data are n or means, SE. Age Range; †HbA1c Reference Range 4.2-5.9%. | |

Each patient had long history of difficult-to treat painful neuropathy and had tried various drugs such as acetaminophen, duloxitine, amitriptyline or gabapentin and had discontinued because the symptoms were unresponsive or due to unacceptable side effects. Eligible subjects included type 1 and type 2 diabetic patients not on any other medications for their neuropathic pain and with stable diabetic control. Exclusion criteria included erratic glycemic control, peripheral vascular disease (PVD) with absent foot pulses, presence of active foot ulceration, treatment with sublingual glyceryl trinitrate, patients on erectile dysfunction drugs, factors affecting the patient's evaluation of pain, and the presence of other causes of peripheral neuropathies. No major changes made for diabetes treatment during the duration of the study.

Patients were assessed neurologically at the beginning of the run in period after which, the patients were randomly allocated to receive the placebo or tapentadol hydrochloride 100 mg or pregabalin 250 mg/gabapentin 250 mg or tapentadol HCl 100 mg + pregabalin 250 mg/gabapentin 250 mg FDC for 4 weeks. A 10-cm visual analog scale (VAS) was recorded biweekly by the patients for pain, where 0 means no pain at all and 10 means the most severe pain ever experienced. The treatment effect was defined to be the difference between the final score and the baseline score on the Lickert scale for each treatment phase.

The objectives of the inventions are met for the fixed dose combination comprising a slow release tapentadol and pregabalin/gabapentin produced statistically significant and clinically meaningful reductions, compared to the mono-therapy using either tapentadol or pregabalin, for the primary efficacy variable in pain intensity associated with diabetic neuropathy. We considered that a clinically significant benefit of using fixed dose slow release tapentadol HCl and pregabalin/gabapentin would be a reduction in the pain score (VAS) of at least 15 % compared to the other treatments.

### Tapentadol and Tramadol Combination, Study III

The Fixed dose combination of slow release tapentadol hydrochloride and tramadol hydrochloride was tested in a human clinical trial. In this study III, 40 patients were used in a clinical trial were suffering from Chronic non-cancer pain (CNCP), defined as pain for longer than 6 months, including neuropathic pain, osteoarthritis, rheumatoid arthritis, fibromyalgia, and back and musculoskeletal pain were included in the clinical trial. No effort was made to segregate the patients based on the type of pain nor did we evaluate the extent of pain relief on the basis of pain type. Those patients with migraines, dental pain, abdominal pains (from chronic pancreatitis, kidney stones, etc.) and ischemic pain from vascular disease were excluded because they are usually not classified as CNCP. Patients with history of addiction (alcohol or drugs) were excluded from the trials. The patient characteristic and the VAS pain score are listed in Table 18 and Table 19, below;

**Table 18**

| **Patient** | | | | |
|---|---|---|---|---|
| **Characteristics** | | | | |
| **Characteristic** | **Tramadol 100 mg** | **Placebo** | **Tapentadol 100 mg** | **Tapentadol 100 mg + Tramadol 50 mg Fixed Dose combination** |
| | **N=10** | **N=10** | **N=10** | **N=10** |
| Mean age +/- SD (y) | 44.2 ±13 | 42.1±11 | 42.5 ± 12 | 41.2 ±12 |
| Sex (M/F) | 23/10 | 20/17 | 22/11 | 19/18 |

**Table 19**

| **VAS Time hours** | | **VAS Pain** | | |
|---|---|---|---|---|
| | | **Score** | | |
| | **Tramadol 100 mg** | **Placebo** | **Tapentadol 100 mg** | **Tapentadol 100 mg + Tramadol 50 mg Fixed Dose combination** |
| | **N=10** | **N=10** | **N=10** | **N=10** |
| 0 | 32.4± 21.2 | 33.9±19.1 | 33.7±18.2 | 31.9±19.1 |
| 3 | 23.1± 20.1 | 32.9±19.1 | 24.8±19.1 | 25.1±17.8 |
| 6 | 18.8 ± 20.6 | 31.9±21.1 | 21.1±22.9 | 22.2±18.6 |
| 9 | 14.9± 17.2 | 30.8±20.3 | 14.2±23.1 | 15.3±20.6 |
| 12 | 13.1± 21.2 | 31.6±18.6 | 11.2±20.9 | 11.3±20.2 |
| 15 | 13.9± 19.2 | 32.1±21.5 | 11.0±16.2 | 9.5±18.9 |
| 18 | 15.4± 21.3 | 32.9±19.7 | 9.5±17.8 | 9.1±22.1 |
| 21 | 14.5± 19.2 | 31.9±18.3 | 10.4±16.2 | 9.3±17.3 |
| 24 | 13.9± 19.2 | 32.8±20.9 | 11.6±16.2 | 10.8±17.5 |

The objectives of the inventions are met for the fixed dose combination comprising a slow release Tapentadol and Tramadol produced statistically significant and clinically meaningful reductions, compared to the mono-therapy using either tapentadol or tramadol, for the primary efficacy variable in pain intensity associated with diabetic neuropathy. We considered that a clinically significant benefit of using fixed dose slow release tapentadol HCl and tramadol (Example 13) would be a reduction in the pain score (VAS) of at least 15 % compared to the other treatments.

### Results

The objectives of the inventions are met by the following results:

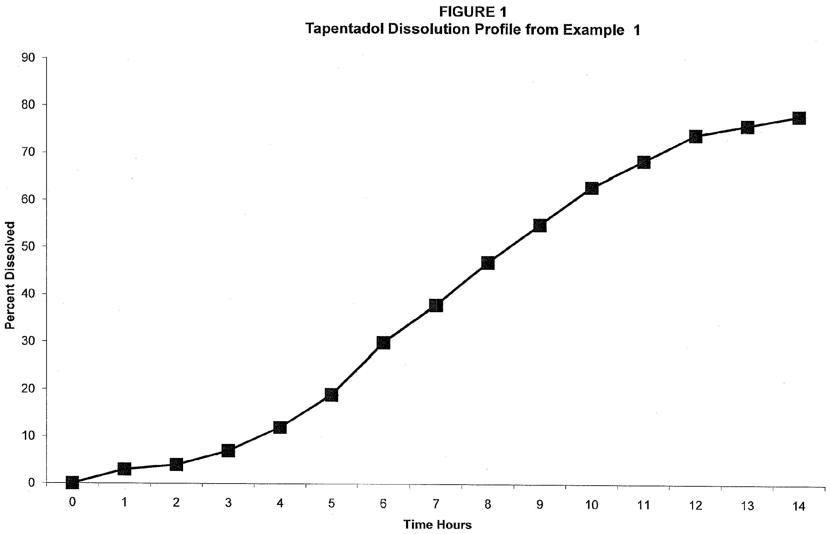

FIGURE 1 illustrates the in vitro dissolution profile of tapentadol HCl in slow release tapentadol HCl 100 mg and naproxen 250 mg tablets formulated according Example 1.

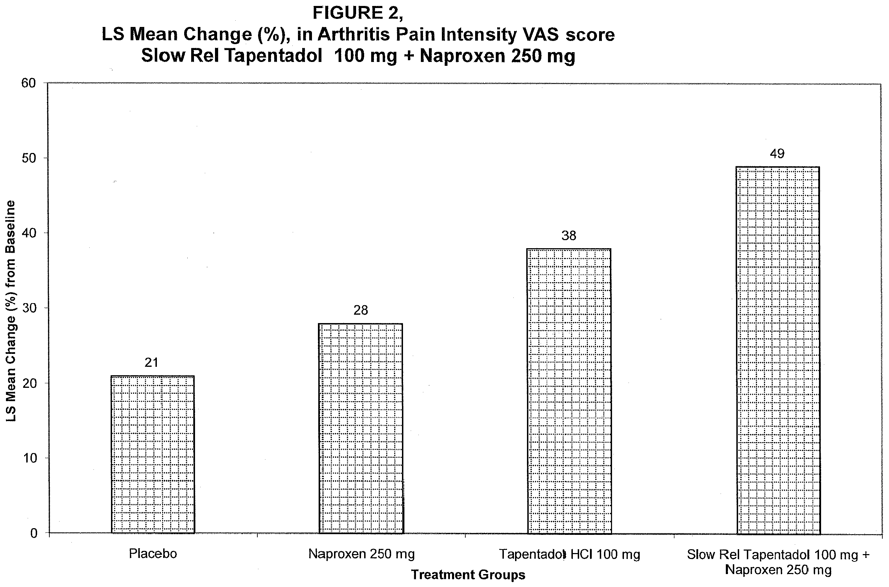

FIGURE 2 compares the LS mean change from baseline in VAS score for the combination drug comprising slow release tapentadol 100 mg and naproxen 250 mg (Example 1) with those of tapentadol and naproxen mono therapies based upon the average of Weeks 1-6. For the primary endpoint (LS Mean change from baseline over 6 weeks), there was a 49.0% change from baseline in the arthritis pain intensity VAS in the slow release tapentadol and naproxen 250 mg fixed dose combination compared 38% for tapentadol, 28% for naproxen and 21% for placebo groups

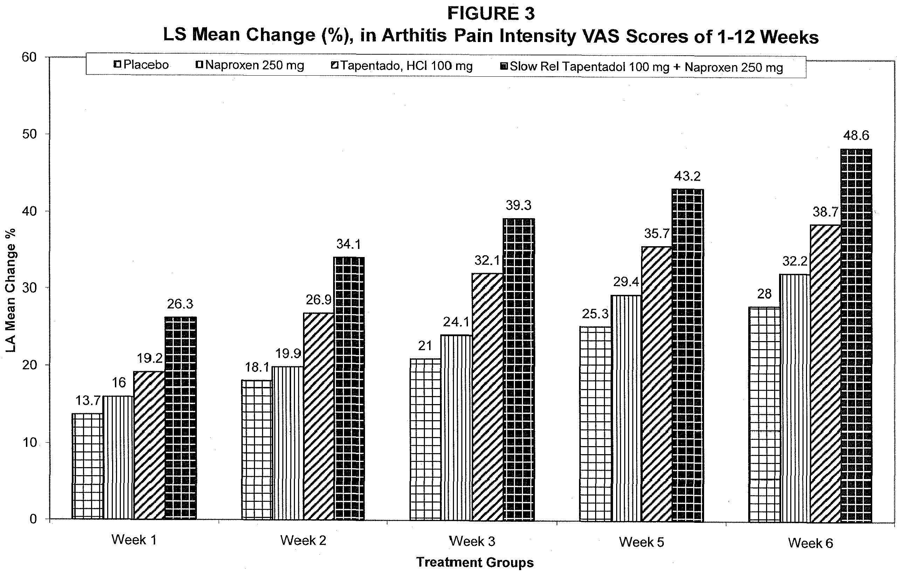

FIGURE 3 shows the weekly LS mean changes from baseline for the four treatment groups. The differences in drug response emerged when patients were receiving either tapentadol HCl 100 mg X 2 or naproxen 250 mg X 2 or the combination of 100 mg tapentadol & 250 mg naproxen (Example 1) X 2 at the very first week. The response to all drugs increased relative placebo from week 1 till week 6. The response to the combination drug was significantly higher than those due to mono therapy treatment with either naproxen or tapentadol.

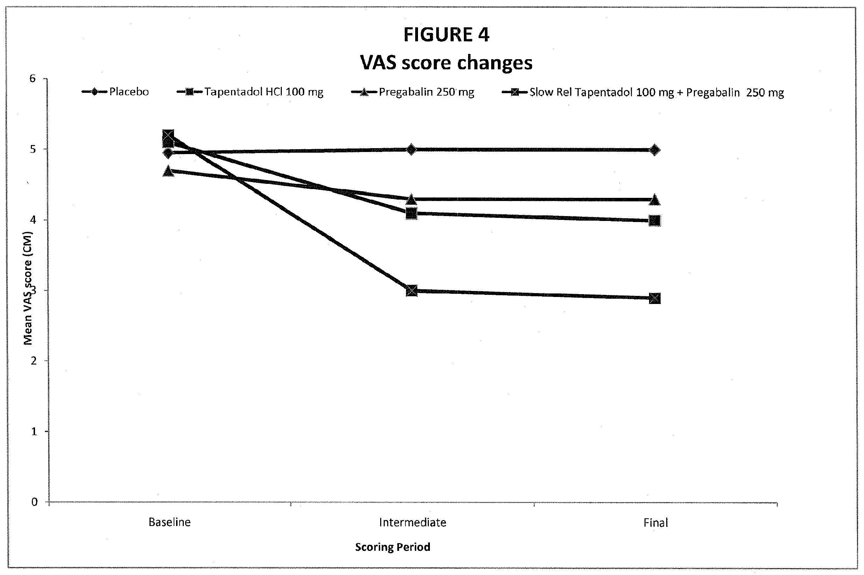

FIGURE 4 shows the mean VAS pain score changes for the four treatments; tapentadol 100 MG, pregabalin 250 MG, and slow release tapentadol 100 MG + pregabalin 250 MG fixed dose combination (Example 10).

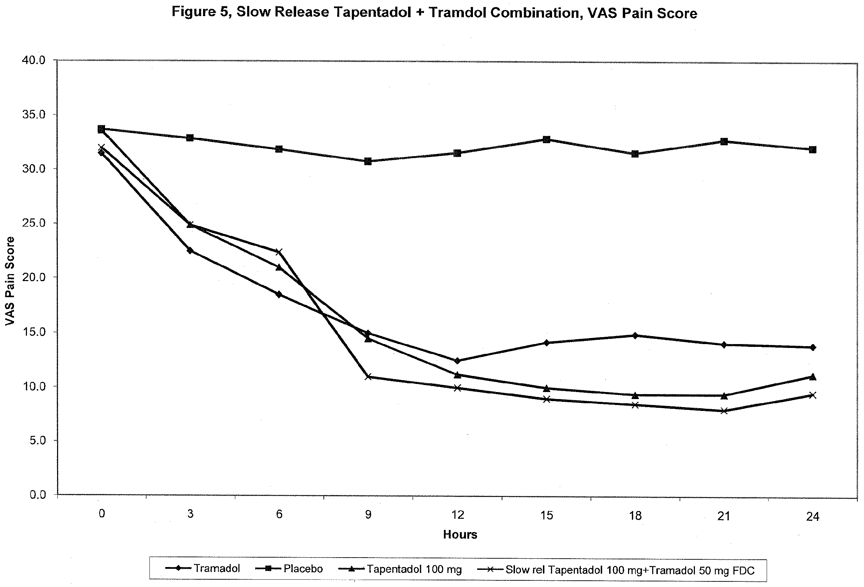

FIGURE 5 shows the mean VAS pain score changes for the three treatments; Tramadol 50 mg, Tapentadol 100 MG, Placebo and Slow Release Tapentadol 100 MG + Tramadol 50 MG (Example 13) fixed dose combination.

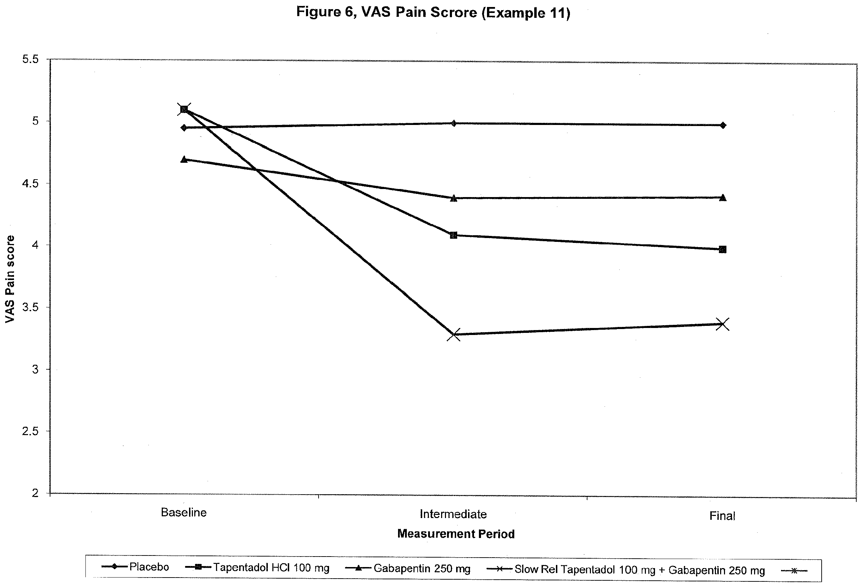

FIGURE 6 shows the mean VAS pain score changes for the four treatments; tapentadol 100 MG, gabapentin 250 MG, and slow release tapentadol 100 plus gabapentin 250 MG fixed dose combination (Example 11).

The abbreviations used herein have their conventional meaning within the chemical and biological arts. All publications, patents, and patent documents cited in the specification are incorporated by reference herein, as though individually incorporated by reference. In the case of any inconsistencies, the present disclosure, including any definitions therein will prevail. The invention has been described with reference to various specific and preferred embodiments and techniques. However, it should be understood that many variations and modifications may be made while remaining within the spirit and scope of the invention.

Further embodiments (emb) of the present invention are:
emb 1. A pharmaceutical composition comprising a slow release tapentadol and at least one pharmaceutically acceptable carrier, and a second active agent, wherein the second active agent is tramadol, a GABA analogues or an NSAID.
emb 2. The pharmaceutical composition of emb 1, wherein the NSAID is Celecoxib, Diclofenac, Diflunisal, Etodolac, Fenoprofen, Flurbirofen, Ibuprofen, Indomethacin, Ketoprofen, Ketorolac, Mefenamic Acid, Meloxicam, Nabumetone, Naproxen, Oxaprozin, Piroxicam, Sulindac, Tolmetin, or a pharmaceutically acceptable salt thereof.
emb 3. The pharmaceutical composition of emb 1 or 2, wherein the GABA analogue is gabapentin, pregabalin, or a pharmaceutically acceptable salt thereof.
emb 4. The pharmaceutical composition of any of emb 1-3, comprising an immediate release coating wherein the immediate release coating comprises the second active agent.
emb 5. The pharmaceutical composition of any of emb 1-4, wherein the composition is a bilayer composition comprising a slow release tapentadol layer, and a layer comprising the second active agent.
emb 6. The pharmaceutical composition of any of emb 1-5, wherein the composition exhibits an in vitro dissolution profile such that after 2 hours, from about 0% to about 30% by weight of tapentadol is released, after 4 hours, from about 5% to about 22% by weight of tapentadol is released, after 6 hours, from about 15% to about 30% by weight of tapentadol is released, and after 8 hours, more than about 40% by weight of tapentadol is released; when measured using the USP Basket Method at 75 rpm in 900 ml 0.1 N HCl at 37° C.
emb 7. The pharmaceutical composition of any of emb 1-6, wherein the composition is a coated tablet wherein the coating comprises at least one water-insoluble, water permeable film-forming polymer, at least one plasticizer and at least one water-soluble polymer, and the second active agent.
emb 8. The pharmaceutical composition of any of emb 1-7, wherein the proportion of the water-insoluble, water-permeable film-forming polymer is about 20% to about 90% of the coating dry weight, the proportion of the plasticizer is about 5% to about 30% of the coating dry weight, and the proportion of the water-soluble polymer is about 10% to about 75% of the coat dry weight.
emb 9. The pharmaceutical composition of emb 8, wherein the water-insoluble, water-permeable film-forming polymer is ethylcellulose, the water-soluble polymer is polyvinylpyrrolidone and the plasticizer is dibutyl sebacate.
emb 10. The pharmaceutical composition of any of emb 1-9, wherein the core comprises a lubricant, a binder, a glidant or any combination thereof.
emb 11. The pharmaceutical composition of any of emb 1-10, wherein the carrier comprises an adjuvant, a preservative, an antioxidant, a thickening agent, a chelating agent, an antifungal agent, an antibacterial agent, an isotonic agent, a flavoring agent, a sweetening agent, an anti-foaming agent, a colorant, a diluent, a moistening agent, a parietal cell activator, or a combination thereof.
emb 12. The pharmaceutical composition of any of emb 1-11, comprising tramadol, Meloxicam, Naproxen, pregabalin or gabapentin.
emb 13. The composition of emb 12, wherein the second active agent is gabapentin and pregabalin or their pharmaceutically acceptable salt.
emb 14. The composition of emb 19, wherein the second active agent is naproxen.
emb 15. The pharmaceutical composition of any of emb 1-14, wherein the tapentadol is present in an amount of from about 5 mg to about 400 mg.
emb 16. The pharmaceutical composition of any of emb 1-14, wherein the GABA analogue is present in an amount of from about 5 mg to about 500 mg.
emb 17. The pharmaceutical composition of any of emb 1-14, wherein an NSAID is present in an amount of from about 5 mg to about 400 mg.
emb 18. A method for treating pain comprising administering to a patient in need thereof a pharmaceutical composition of any of emb 1-17, comprising a slow release tapentadol and a pharmaceutically acceptable carrier, and a second active agent, wherein the second active agent is tramadol, a GABA analogues or an NSAID.
emb 19. The method of emb 18, wherein the NSAID is Celecoxib, Diclofenac, Diflunisal, Etodolac, Fenoprofen, Flurbirofen, Ibuprofen, Indomethacin, Ketoprofen, Ketorolac, Mefenamic Acid, Meloxicam, Nabumetone, Naproxen, Oxaprozin, Piroxicam, Sulindac and Tolmetin or their pharmaceutically acceptable salt.
emb 20. The method of emb 18, wherein the GABA analogue is gabapentin and pregabalin or their pharmaceutically acceptable salt.
emb 21. The method of any of emb 18-20, wherein the pain is neuropathic pain, osteoarthritis, rheumatoid arthritis, fibromyalgia, and back, musculoskeletal pain, ankylosing spondylitis, juvenile rheumatoid arthritis, diabetic neuropathy, migraines, dental pain, abdominal pains, ischemic pain, postoperative pain or because of an anesthetic or surgical contrition.
emb 22. A pharmaceutical kit comprising a formulation of slow release tapentadol, and at least one pharmaceutically acceptable carrier, and a second active agent, of any of emb 1-17, wherein the second active agent is selected from tramadol, a GABA analogue and an NSAID.
emb 23. Use of a composition of any of emb 1-17, to prepare a medicament for treatment of pain.
emb 24. The use of emb 23, wherein the NSAID is naproxen.
emb 25. The use of emb 23, wherein the GABA analogue is pregabalin or gabapentin.

## Claims

1. A pharmaceutical composition comprising a slow release tapentadol and at least one pharmaceutically acceptable carrier, and a second active agent, wherein the second active agent is tramadol, a GABA analogues or an NSAID.

2. The pharmaceutical composition of claim 1, wherein the NSAID is Celecoxib, Diclofenac, Diflunisal, Etodolac, Fenoprofen, Flurbirofen, Ibuprofen, Indomethacin, Ketoprofen, Ketorolac, Mefenamic Acid, Meloxicam, Nabumetone, Naproxen, Oxaprozin, Piroxicam, Sulindac, Tolmetin, or a pharmaceutically acceptable salt thereof.

3. The pharmaceutical composition of claim 1 or 2, wherein the GABA analogue is gabapentin, pregabalin, or a pharmaceutically acceptable salt thereof.

4. The pharmaceutical composition of any of claims 1-3, comprising an immediate release coating wherein the immediate release coating comprises the second active agent.

5. The pharmaceutical composition of any of claims 1-4, wherein the composition is a bilayer composition comprising a slow release tapentadol layer, and a layer comprising the second active agent.

6. The pharmaceutical composition of any of claims 1-5, wherein the composition exhibits an in vitro dissolution profile such that after 2 hours, from about 0% to about 30% by weight of tapentadol is released, after 4 hours, from about 5% to about 22% by weight of tapentadol is released, after 6 hours, from about 15% to about 30% by weight of tapentadol is released, and after 8 hours, more than about 40% by weight of tapentadol is released; when measured using the USP Basket Method at 75 rpm in 900 ml 0.1 NHCl at 37°C.

7. The pharmaceutical composition of any of claims 1-6, wherein the composition is a coated tablet wherein the coating comprises at least one water-insoluble, water permeable film-forming polymer, at least one plasticizer and at least one water-soluble polymer, and the second active agent.

8. The pharmaceutical composition of any of claims 1-7, wherein the proportion of the \water-insoluble, water-permeable film-forming polymer is about 20% to about 90% of the coating dry weight, the proportion of the plasticizer is about 5% to about 30% of the coating dry weight, and the proportion of the water-soluble polymer is about 10% to about 75% of the coat dry weight.

9. The pharmaceutical composition of claim 8, wherein the water-insoluble, water-permeable film-forming polymer is ethylcellulose, the water-soluble polymer is polyvinylpyrrolidone and the plasticizer is dibutyl sebacate.

10. The pharmaceutical composition of any of claims 1-9, wherein the core comprises a lubricant, a binder, a glidant or any combination thereof.

11. The pharmaceutical composition of any of claims 1-10, wherein the carrier comprises an adjuvant, a preservative, an antioxidant, a thickening agent, a chelating agent, an antifungal agent, an antibacterial agent, an isotonic agent, a flavoring agent, a sweetening agent, an anti-foaming agent, a colorant, a diluent, a moistening agent, a parietal cell activator, or a combination of thereof.

12. The pharmaceutical composition of any of claims 1-11, tramadol, Meloxicam, Naproxen, pregabalin or gabapentin.

13. The composition of claim 12, wherein the second active agent is gabapentin and pregabalin or their pharmaceutically acceptable salt.

14. The composition of claim 19, wherein the second active agent is naproxen.

15. The pharmaceutical composition of any of claims 1-14, wherein the tapentadol is present in an amount of from about 5 mg to about 400 mg.

16. The pharmaceutical composition of any of claims 1-14, wherein the GABA analogue is present in an amount of from about 5 mg to about 500 mg.

17. The pharmaceutical composition of any of claims 1-14, wherein an NSIAD is present in an amount of from about 5 mg to about 400 mg.

18. A method for treating pain comprising administering to a patient in need thereof a pharmaceutical composition of any of claims 1-17, comprising a slow release tapentadol and a pharmaceutically acceptable carrier, and a second active agent, wherein the second active agent is tramadol, a GABA analogues or an NSAID.

19. The method of claim 18, wherein the NSAID is Celecoxib, Diclofenac, Diflunisal, Etodolac, Fenoprofen, Flurbirofen, Ibuprofen, Indomethacin, Ketoprofen, Ketorolac, Mefenamic Acid, Meloxicam, Nabumetone, Naproxen, Oxaprozin, Piroxicam, Sulindac and Tolmetin or their pharmaceutically acceptable salt.

20. The method of claim 18, wherein the GABA analogue is gabapentin and pregabalin or their pharmaceutically acceptable salt.

21. The method of any of claims 18-20, wherein the pain is neuropathic pain, osteoarthritis, rheumatoid arthritis, fibromyalgia, and back, musculoskeletal pain, ankylosing spondylitis, juvenile rheumatoid arthritis, diabetic neuropathy, migraines, dental pain, abdominal pains, ischemic pain, postoperative pain or because of an anesthetic or surgical contrition.

22. A pharmaceutical kit comprising a formulation of slow release tapentadol, and at least one pharmaceutically acceptable carrier , and a second active agent, of any of claims 1-17, wherein the second active agent is selected from tramadol, a GABA analogue and an NSAID.

23. Use of a composition of any of claims 1-17, to prepare a medicament for treatment of pain.

24. The use of claim 23, wherein the NSAID is naproxen.

25. The use of claim 23, wherein the GABA analogue is pregabalin or gabapentin.
